(19) Europäisches Patentamt
European Patent Office
Office européen des brevets

(11) EP 3 223 792 B1

(12) # FASCICULE DE BREVET EUROPEEN

(45) Date de publication et mention de la délivrance du brevet:
**05.09.2018 Bulletin 2018/36**

(21) Numéro de dépôt: **15802076.8**

(22) Date de dépôt: **30.11.2015**

(51) Int Cl.:
***A61K 8/73*** (2006.01) ***A61Q 19/08*** (2006.01)
***C08B 37/00*** (2006.01)

(86) Numéro de dépôt international:
**PCT/EP2015/078001**

(87) Numéro de publication internationale:
**WO 2016/083614 (02.06.2016 Gazette 2016/22)**

(54) **POLYSACCHARIDE GREFFÉ PHOTOACTIF ET SON UTILISATION EN COSMÉTIQUE**

PHOTOAKTIVES GEPFROPFTES POLYSACCHARID UND VERWENDUNG DAVON IN DER KOSMETIK

PHOTOACTIVE GRAFTED POLYSACCHARIDE AND USE THEREOF IN COSMETICS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2014 FR 1461656**

(43) Date de publication de la demande:
**04.10.2017 Bulletin 2017/40**

(73) Titulaire: **L'Oréal**
**75008 Paris (FR)**

(72) Inventeur: **GREAVES, Andrew**
**77700 Magny-le-Hongre (FR)**

(74) Mandataire: **Kromer, Christophe**
**L'Oréal**
**Service DIPI**
**9 Rue Pierre Dreyfus**
**92110 Clichy (FR)**

(56) Documents cités:
**FR-A1- 2 991 583**

- **AKIRA TAKAHASHI ET AL: "In Situ Cross-Linkable Hydrogel of Hyaluronan Produced via Copper-Free Click Chemistry", BIOMACROMOLECULES, vol. 14, no. 10, 14 octobre 2013 (2013-10-14), pages 3581-3588, XP055197139, ISSN: 1525-7797, DOI: 10.1021/bm4009606**
- **LAVILLE MAXIME ET AL: "Polysaccharide-covered nanoparticles with improved shell stability using click-chemistry strategies", CARBOHYDRATE POLYMERS, APPLIED SCIENCE PUBLISHERS, LTD. BARKING, GB, vol. 93, no. 2, 22 décembre 2012 (2012-12-22), pages 537-546, XP028996623, ISSN: 0144-8617, DOI: 10.1016/J.CARBPOL.2012.11.050**
- **Anonymous: "Photoreactive Crosslinker Chemistry | Life Technologies", , 24 juin 2015 (2015-06-24), XP055198085, Extrait de l'Internet: URL:https://www.lifetechnologies.com/de/de/home/life-science/protein-biology/protein-biology-learning-center/protein-biology-resource-library/pierce-protein-methods/photoreactive-crosslinker-chemistry.html [extrait le 2015-06-24]**
- **GEORGINA K. SUCH ET AL: "Synthesis and functionalization of nanoengineered materials using click chemistry", PROGRESS IN POLYMER SCIENCE, vol. 37, no. 7, 1 juillet 2012 (2012-07-01), pages 985-1003, XP055182159, ISSN: 0079-6700, DOI: 10.1016/j.progpolymsci.2011.12.002**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé pour atténuer les rides de la peau utilisant une composition comprenant un polymère polysaccharide greffé d'un groupement photoactif particulier, et l'exposition à la lumière de la peau traitée.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge.

Il est connu de traiter ces signes du vieillissement en utilisant des compositions cosmétiques ou dermatologiques contenant des actifs capables de lutter contre le vieillissement, tels que les α-hydroxy-acides, les β-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés, conduisant rapidement à un lissage des rides et ridules et à la disparition des marques de fatigue.

Les inventeurs ont découvert que l'application topique sur la peau d'un polymère polysaccharide greffé de groupements photoactifs de type azide ou diazirine, combinée à l'exposition de la peau traitée avec un rayonnement lumineux, forme un film présentant notamment un effet tenseur sur la peau amélioré et permet ainsi d'atténuer les rides de la peau de façon rapide. Le film obtenu présente une bonne résistance à l'eau et à la sueur. L'effet tenseur du film sur la peau présente également une bonne résistance à l'eau et donc une bonne rémanence à l'eau, ainsi qu'à la sueur, au sébum. Le dépôt du polymère obtenu après exposition à la lumière est également résistant aux sollicitations mécaniques de la peau (engendrés par les mouvements de la peau).

De façon plus précise, la présente invention a pour objet un procédé, notamment cosmétique, de soin de la peau, plus particulièrement de la peau du visage, en particulier de la peau ridée, comprenant :

(i) une étape consistant à appliquer sur la peau une composition, notamment cosmétique, comprenant, dans un milieu physiologiquement acceptable, un polymère polysaccharide greffé de groupements photoactifs de type azide ou diazirine de formule (I) tel que défini ci-après ;

(ii) une étape consistant à exposer la peau traitée à un rayonnement lumineux, de préférence pendant au moins 5 secondes. Cette étape peut être répétée plusieurs fois pendant la journée.

Le procédé selon l'invention est en particulier destiné à lisser la peau humaine du visage et/ou du corps et/ou à diminuer ou effacer les signes du vieillissement cutané, en particulier à réduire ou effacer les rides et/ou les ridules de la peau.

On entend par « agent tenseur » des composés susceptibles d'avoir un effet tenseur apparent, c'est-à-dire de lisser la peau et réduire, voire faire disparaître, de façon rapide, voire immédiate, les rides et les ridules.

L'effet tenseur peut être caractérisé par un test *in vitro* de rétractation tel que décrit à l'exemple 1.

Le polymère polysaccharide greffé de groupements photoactifs de type azide ou ou diazirine répond à la formule (I) suivante :

$$PS\text{-}(O\text{-}CO\text{-}\text{-}L\text{-}X)_a(OH)_b$$

dans laquelle PS désigne le squelette de base du polysaccharide portant les groupes hydroxyle ;
L est un groupe divalent hydrocarboné comprenant de 1 à 20 atome de carbone, de préférence de 2 à 10 atomes de carbone, linéaire, ramifié ou cyclique, saturée ou insaturée, et pouvant être interrompu par un ou plusieurs hétéroatomes non adjacents choisis parmi le soufre, l'oxygène ou groupements -NH-, -COO-, -CO-NH- ,-O-CO-NH-,-NH-CO-NH-, ledit groupement divalent étant éventuellement substitué par un ou plusieurs groupes choisi parmi les groupes hydroxyle, amine, thiol, acide carboxylique, amide, cyano, acyl ($C_1$-$C_4$)amino ;
X désigne un groupe photoactif de type azide ou diazirine
a désigne le taux de groupe OH substitué par le groupe -CO-L-X
b désigne le taux de groupe OH libres non substitués
a étant compris entre 0,02 et 0,5 ; b étant compris entre 0,5 et 0,98 ;
a + b = 1

Par exemple lorsque a = b = 0,5, cela signifie que la moitié des groupes hydroxyle du polysaccharide sont greffés par le groupement -CO-L-X et l'autre moitié des groupes hydroxyles ne sont pas greffés, correspondant au polymère greffé de formule

$$PS\text{-}(O\text{-}CO\text{-}\text{-}L\text{-}X)_{0,5}(OH)_{0,5}$$

De préférence a est compris entre 0,02 et 0,4 ; b est compris entre 0,6 et 0,98. Préférentiellement, a est compris entre 0,02 et 0,3 ; b est compris entre 0,7 et 0,98. Plus préférentiellement, a est compris entre 0,04 et 0,2 ; b est compris entre 0,8 et 0,96. Mieux, a est compris entre 0,04 et 0,15 ; b est compris entre 0,85 et 0,96.

Le polysaccharide peut comprendre en plus des groupes hydroxyles des groupes additionnels tels que acide carboxylique, amino (-$NH_2$), aminoacétyle (-NHAc). Ces groupes additionnels font partie du squelette de base du polysaccharide portant les groupes hydroxyle.

Le polysaccharide peut comprendre un ou plusieurs motifs de base choisi parmi l'acide uronique, l'acide glucuronique, l'acide manuronique et de préférence l'acide uronique. Le polysaccharide utilisé selon l'invention peut être choisi parmi l'acide hyaluronique, le chondroitine, le chondroitine sulfate, l'acide alginique, l'héparine, l'héparine sulfate, la gomme de xanthane, le dextran, la cellulose.

De préférence, le polysaccharide est l'acide hyaluronique ou l'acide alginique. Préférentiellement le polysaccharide est l'acide hyaluronique.

L'acide hyaluronique est un glycosaminoglycane linéaire composé d'unités répétitives de D-acide glucuronique et de N-acetyl-D-glucosamine liés entre eux par des liaisons glycosidiques alternées beta-1,4 et beta-1,3.

Avantageusement, le polysaccharide greffé a un poids moléculaire moyen en poids allant de 5000 à 1 000 000 daltons, de préférence allant de 10 000 à 500 000 daltons, et encore plus préférentiellement allant de 15000 à 350 000 daltons.

Le poids moléculaire peut être notamment déterminé par chromatographie phase liquide, éluant chlorure de sodium 0,1 M et 330 mg/l d'azoture de sodium dans l'eau, étalon dextran, détecteurs Réfractomètre OPTILAB T-Rex de WYATT et Diffusion de lumière DAWN-HELEOS II - WYATT.

De préférence, le polysaccharide greffé est un acide hyaluronique greffé comprenant des motifs greffés de formule suivante :

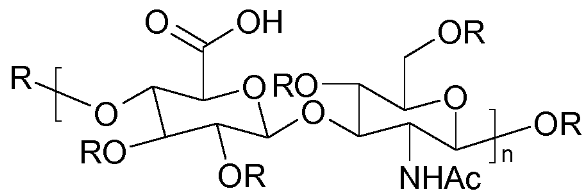

dans laquelle R représente indépendamment H ou un groupement -CO-L-X, L et X ayant les significations décrites précédemment, étant entendu que le polysaccharide greffé a un taux de greffage allant de 2 à 50 %.

n étant notamment tel que le polymère greffé a un poids moléculaire tel que défini précédemment.

De préférence, le groupe L est choisi parmi les groupes suivants :

| | | |
|---|---|---|
| 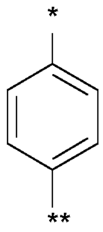 | 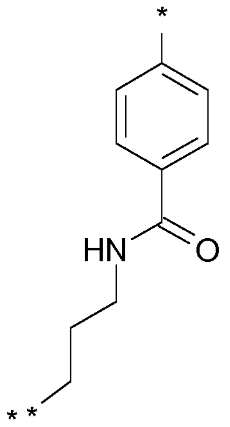 | 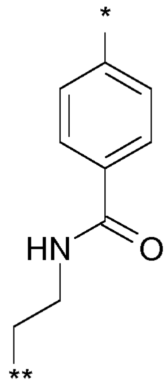 |
| 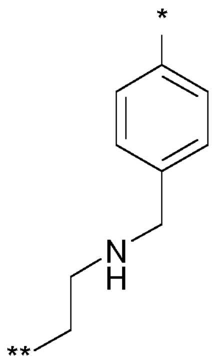 | $*-CH_2-[CH_2]_m-**$<br>m = 1-3 | 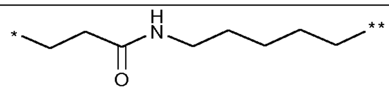 |

*représentant la liaison avec le groupement photoactif X

** représente la liaison avec le groupe ester lié au polymère PS (groupe PS-O-CO-)

Le groupe photoactif X peut être choisi parmi les groupes suivants :

| | | | |
|---|---|---|---|
| $*-N_3$ | 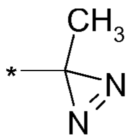 | 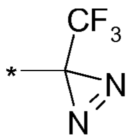 | 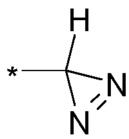 |

Comme exemple de groupement X-L- , on peut citer les groupements suivants :

| | | |
|---|---|---|
| 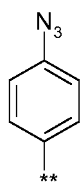<br>(i) | 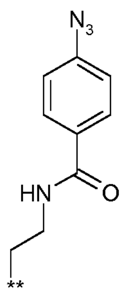<br>(ii) | 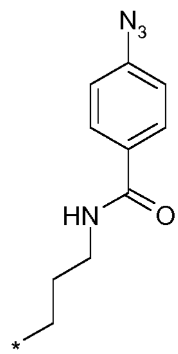<br>(iii) |

(suite)

| | | |
|---|---|---|
| $N_3$-$CH_2$-$CH_2$-** (iv) | 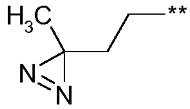 (v) | 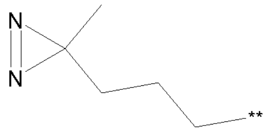 (vi) |
| 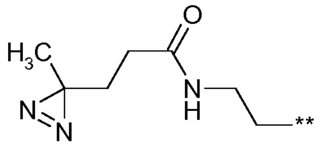 (vii) | 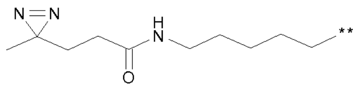 (viii) | 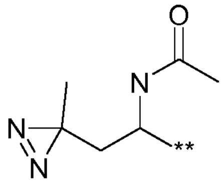 (ix) |
| 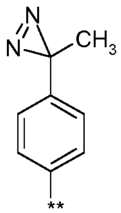 (x) | 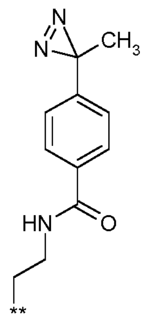 (xi) | 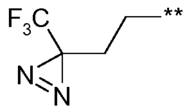 (xii) |
| 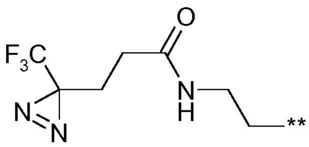 (xiii) | 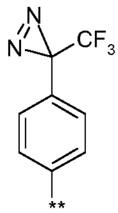 (xiv) | 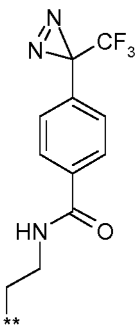 (xv) |
| ** représente la liaison avec le groupe ester lié au polymere PS (groupe PS-O-CO-) | | |

Les groupements (i) et (v) sont préférés.

Les polysaccharides greffés utilisés selon l'invention peuvent être préparés notamment par activation d'un acide carboxylique X-L-COOH (A) avec un carbodiimide (B) (par exemple l'hydrochlorure de 1-éthyl-3-(3-diméthylaminopropyl) carbodiimide) - pour former l'acide activé (C) - puis greffage sur les groupements hydroxyle du polysaccharide (schéma de synthèse I). La réaction peut également s'effectuer en présence d'un deuxième activateur d'acide carboxylique tel que les N-hydroxybenzotriazoles et les N-hydroxysuccinimides, en particulier le N-hydroxysuccinimide (D) (schéma de synthèse II) pour former un intermédiaire (E) avant le greffage sur les groupes hydroxyle du polysaccharide. Le deuxième activateur permet de stabiliser l'acide carboxylique activé, de limiter l'hydrolyse l'acide carboxylique activé et également d'augmenter le taux de greffage sur le polysaccharide.

La réaction peut se faire dans un solvant aprotique ou protique. De préférence la réaction est effectuée dans l'eau, à un pH compris entre 4 et 9 et préférablement entre 5 et 7. La réaction est de préférence effectuée à une température comprise entre 5 et 80 °C et préférentiellement à la température ambiante (25 °C).

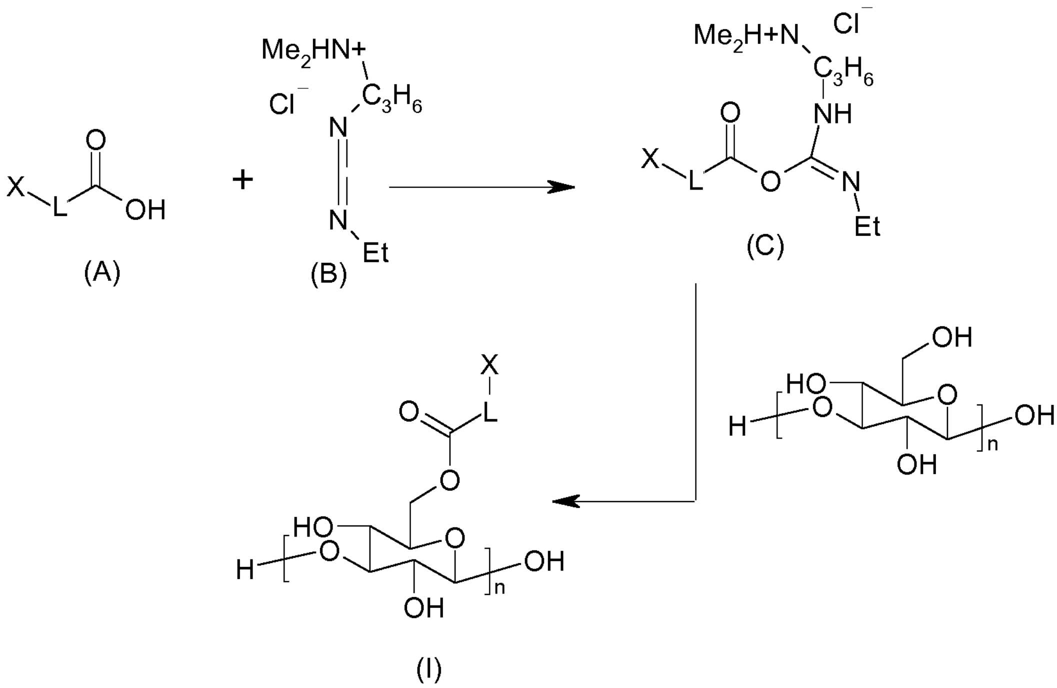


Schéma de synthèse I

L'utilisation de N-hydroxysuccimide dans le procédé de synthèse conduit au schéma de réaction décrit ci-après :

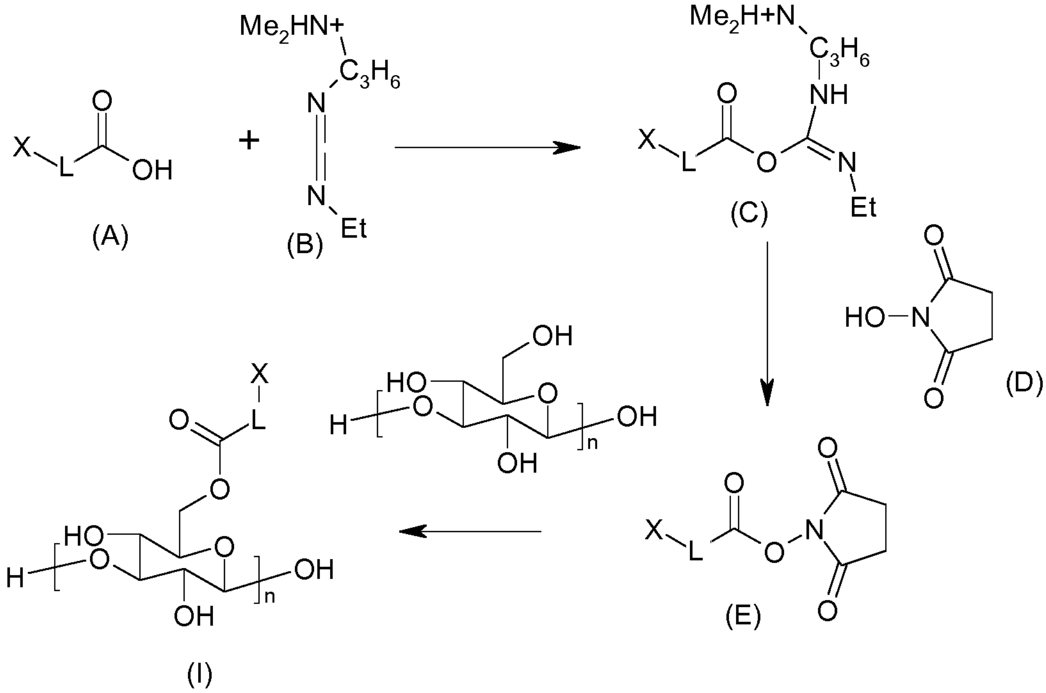


Schéma de synthèse II

Da façon simplifiée, le schéma de réaction est le suivant :

$$\underset{}{\text{PS-OH}} + \underset{(C)}{\text{A-O-CO-L-X}} \longrightarrow \underset{(I)}{\text{PS(OCO-L-X)}_a\text{(OH)}_b}$$

A représentant un groupement activateur d'acide carboxylique comme par exemple les carbodiimides. Certains composés X-L-COO-A sont disponibles tels que les composés décrits ci-après :

| | |
|---|---|
| Ester de l'acide 4-[3-(trifluoromethyl)diazirin-3-yl]benzoïque dede N-hydroxysuccinimide de la société Toronto Research Chemicals | 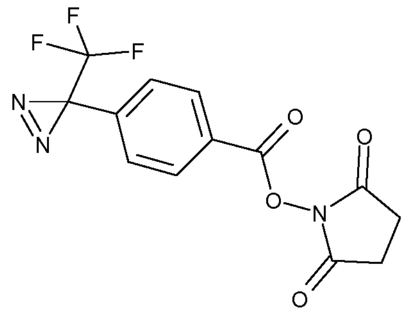 |
| Succinimidyl-diazirine (SDA de la société ThermoScientific) | 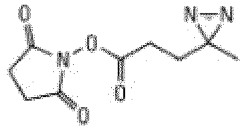 |
| Sulfo- Succinimidyl-diazirine (Sulfo-SDA de la société ThermoScientific) | 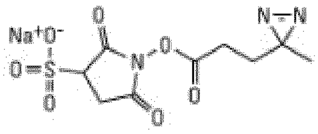 |
| 6-[4,4-azipentanamido]hexanoate de succinimidyle (LC-SDA de la société ThermoScientific) | 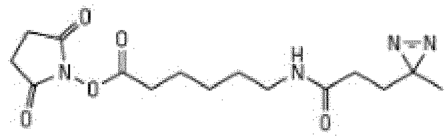 |
| 6-[4,4-azipentanamido]hexanoate de sulfosuccinimidyle (Sulfo-LC-SDA de la société ThermoScientific) | 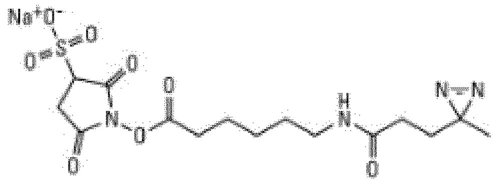 |

Certaines des acides carboxyliques de départ (X-L-COOH) et non-activés sont disponibles dans la commerce tel que ceux cités dans le tableau ci-dessous :

| | |
|---|---|
| 4-[3-(trifluoromethyl)-3h-diazirin-3-yl]benzoic acid de la société TCI | 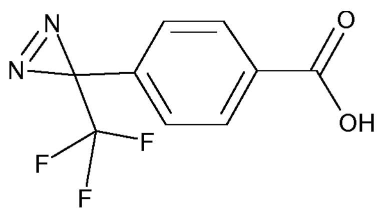 |
| alpha-(acetylamino)-3-methyl-3h-diazirine-3-propanoic acid de la société ChemStep | 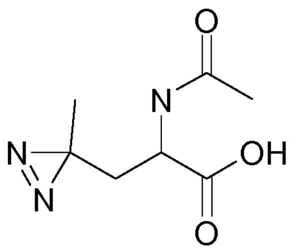 |

(suite)

| | |
|---|---|
| 4-(3-methyl-3h-diazirin-3-yl)butanoic acid de la société FineChemie & Pharma | 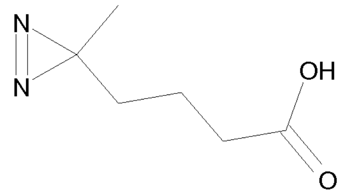 |
| 4-[3-(trifluoromethyl)-3h-diazirin-3-yl]-benzenepropanoic acid de la société Dalton Pharma | 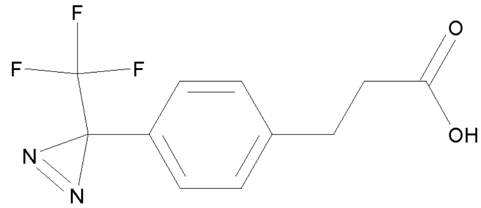 |

Avantageusement, le polymère polysaccharide greffé a un taux de greffage en groupements photoactifs allant de 2 à 50 % de préférence allant de 2 à 40 %, de préférence allant de 2 à 30 %, préférentiellement allant de 4 à 20 %, et mieux allant de 4 à 15 %. Le taux de greffage correspond au pourcentage de groupes OH du polysaccharide qui sont greffés par un groupement photoactif -CO-L-X.

A titre d'exemple un taux de greffage de 50 % correspond à la moitié des groupes OH du polysaccharide greffé par un groupement photoactif -CO-L-X.

Des polymères d'acide hyaluronique à groupement ester issu de l'acide 4-azidobutyrique utilisés pour former des hydrogels injectables sont décrits dans l'article « In situ cross-linkable hydrogel of hyaluronan produced via copper-free click chemistry » Akira Takahashi et al, Biomacromolecules, 2013, 14, pages 3581-3588.

Des polymères de dextran à groupement ester issu de l'acide 4-azidohexanoïque utilisés comme tensioactifs sont décrits dans l'article « Polysaccharide-covered nanoparticles with improved shell stability using click-chemistry stratégies » Laville M et al, Carbohydrate polymers 93 (2013) 537-546.

Les polysaccharides greffés de formule (I) définis précédemment sont des composés nouveaux à l'exception des composés (I) pour lesquels :

PS est le dextran et -L-X = $-(CH_2)_5-N_3$
PS est l'acide hyaluronique et -L-X = $-(CH_2)_3-N_3$

L'invention a donc pour objet ces composés nouveaux de formule (I).

L'invention a aussi pour objet une composition comprenant dans un milieu physiologiquement acceptable un polysaccharide greffé (I) tel que défini précédemment.

La composition selon l'invention est généralement adaptée à une application topique sur la peau et comprend donc généralement un milieu physiologiquement acceptable, c'est-à-dire un milieu compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

Le polysaccharide greffé (I) peut être présent dans la composition selon l'invention en une teneur allant de 0,1 à 10 % en poids, par rapport au poids total de la composition, de préférence allant de 0,5 % à 10 % en poids, et préférentiellement allant de 1 à 8 % en poids, et plus préférentiellement allant de 1 % à 6 % en poids.

La composition selon l'invention peut se présenter sous toutes les formes galéniques classiquement utilisées pour une application topique et notamment sous forme de dispersions du type lotion ou gel aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E ou d'un gel aqueux.

Avantageusement, la composition utilisée selon l'invention comprend de l'eau, notamment en une teneur pouvant aller de 10 à 99 % en poids, par rapport au poids total de la composition, et de préférence allant de 50 à 99 % en poids.

La composition utilisée selon l'invention peut contenir en outre contenir un ou plusieurs adjuvants couramment utilisés dans le domaine cosmétique, tels que des émulsionnants, des conservateurs, des séquestrants, des parfums, des épaississants, des huiles, des cires, des polymères filmogènes.

Bien entendu, l'homme du métier veillera à choisir ce ou ces éventuels composés additionnels et/ou leur quantité de manière telle que les propriétés anti-rides de la composition selon l'invention ne soient pas, ou substantiellement pas, altérées par l'adjonction envisagée.

Avantageusement, pour le procédé selon l'invention, il est possible d'appliquer 0,001 à 0,5 g de composition cosmétique comprenant le polymère polysaccharide à motif acide uronique greffé, notamment 0,005 à 0,1 g de composition, par $cm^2$ de peau.

Le procédé selon l'invention comprend également une étape consistant à exposer la peau à un rayonnement lumineux présentant de préférence une longueur d'ondes comprise entre 360 et 600 nm.

Il est possible d'effectuer cette étape consistant à appliquer un rayonnement lumineux avant, après ou en même temps (simultanément) que l'étape consistant à appliquer la composition comprenant le polymère polysaccharide greffé. De préférence, les deux étapes ont lieu simultanément.

Préférentiellement, dans un premier temps, on applique sur la peau la composition comprenant le polymère polysaccharide à motif acide uronique greffé, puis dans un second temps, on applique un rayonnement lumineux sur la peau. Il est possible de procéder à une étape de rinçage, par exemple à l'eau, de la peau entre chaque étape du procédé.

De préférence, le rayonnement lumineux utilisé dans le procédé selon l'invention présente une longueur d'ondes comprise entre 400 et 480 nm.

Le rayonnement lumineux présente de préférence une fluence (quantité d'énergie par unité de surface) allant de 0,1 à 100 $J/cm^2$, de préférence allant de 1 à 10 $J/cm^2$.

Le rayonnement lumineux peut être une lumière continue ou non continue.

Le rayonnement lumineux peut être la lumière naturelle (lumière du jour).

Le rayonnement lumineux peut être généré par un dispositif, tel que les lampes d'arc telles que les lampes à xénon et les lampes à mercure; les lampes à fluorescence; les lampes à incandescence telles que les halogènes; les LEDs et les lasers.

On peut notamment citer les goLITE BLU de la société Philips, la lampe l'Energylight HF 3319/01 de la société Philips, les lampes Dayvia White et Messa de la société Solvital, la lampe Lumino Plus de la société Lanaform, la lampe medibeam de la société Medibeam, la lampe M-LED 01 de la société Meimed, la lampe Lifemax light pod de la société Lifemax, la lampe Lite-Pad de la société Reicorp, et la lampe Camag Box 3 (4x8W) de la société Camag.

La durée d'exposition au rayonnement lumineux de la peau traitée et apporté par un dispositif est de préférence d'au moins 5 secondes, De préférence cette durée d'exposition peut aller de 10 secondes à 15 minutes, notamment entre 15 secondes et 10 minutes, encore mieux entre 20 secondes et 5 minutes, quel que soit l'ordre des étapes (l'une avant l'autre ou simultanées).

A titre d'exemple, en cas d'application simultanée du rayonnement lumineux apporté par un dispositif et de la composition comprenant le polymère polysaccharide greffé, la durée d'exposition à la lumière peut avantageusement aller de 5 secondes à 15 minutes. Il est possible de procéder à un rinçage de la composition.

A titre d'exemple, en cas d'application de la composition selon l'invention, puis d'exposition au rayonnement lumineux apporté par un dispositif, la durée d'exposition à la lumière peut avantageusement être comprise entre 5 secondes et 15 minutes. Il est possible de laisser poser la composition utilisée selon l'invention pendant une durée de 1 seconde à 3 heures, avant de procéder à l'étape d'application du rayonnement lumineux. Il est possible de procéder à un rinçage de la composition, après l'étape d'exposition au rayonnement lumineux.

La durée d'exposition de la peau traitée à la lumière du jour comme rayonnement lumineux est de préférence d'au moins 3 minutes, De préférence cette durée d'exposition peut aller de 3 minutes à 12 heures, notamment entre 5 minutes et 90 minutes, encore mieux entre 10 minutes et 30 minutes, quel que soit l'ordre des étapes (l'une avant l'autre ou simultanées).

A titre d'exemple, en cas d'application simultanée de la lumière du jour et de la composition comprenant le polysaccharide greffé, la durée d'exposition à la lumière peut avantageusement aller de 3 minutes à 12 heures. Il est possible de procéder à un rinçage de la composition.

A titre d'exemple, en cas d'application de la composition comprenant le polysaccharide greffé, puis d'exposition à la lumière du jour, la durée d'exposition à la lumière peut avantageusement être comprise entre 3 minutes à 12 heures. Il est possible de laisser poser la composition selon l'invention pendant une durée de 1 seconde à 3 heures, avant de procéder à l'étape d'exposition au rayonnement lumineux.

Il est possible de procéder à un rinçage de la composition, après l'étape d'exposition au rayonnement lumineux, mais cela n'est pas obligatoire.

L'étape d'exposition au rayonnement lumineux peut être répétée plusieurs fois pendant la journée.

L'application de la composition cosmétique utilisée selon l'invention se fait selon les techniques habituelles, par exemple par application (notamment de crèmes, de gels, de sérums, de lotions) sur la peau destinée à être traitée,

en particulier la peau du visage et/ou du cou, notamment la peau du contour de l'oeil. Dans le cadre de ce procédé, la composition peut être, par exemple, une composition de soin.

L'invention va maintenant être décrite en référence aux exemples suivants donnés à titre illustratif et non limitatif. Les teneurs sont exprimées en pourcentage pondéral.

**Exemple de synthèse 1 (polymère 1):** Acide hyaluronique fonctionnalisé à 6 % par des groupes diazirine

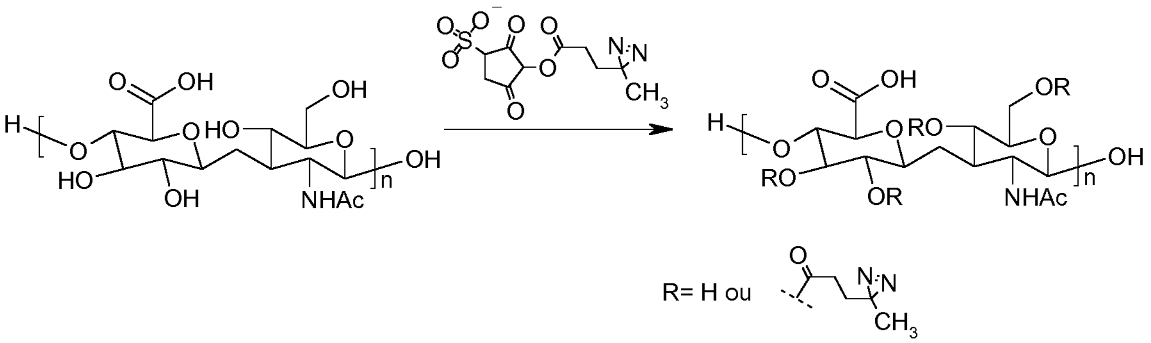


210 mg d'acide hyaluronique (Hyacare® 50 de chez Evonik) a été dissous dans 4,2 ml d'eau distillée dans un ballon recouvert d'une feuille d'aluminium pour empêcher l'exposition à la lumière. 50 mg de 4,4'-azipentanoate de succinimidyle (sulfo-SDA de la société ThermoScientific) a été ajouté, sous agitation à température (5°C). le mélange réactionnel a été agité pendant 24 h en laissant la température augmenter à 22°C et en maintenant le pH entre 6 et 7 par addition d'une solution aqueuse d'hydroxyde de sodium 0,5 M et de solution aqueuse d'acide chlorohydrique 0,5 M.

210 mg d'acide hyaluronique (Hyacare® 50 de chez Evonik) a été dissous dans 4,2 ml d'eau distillée dans un ballon recouvert d'une feuille d'aluminium pour empêcher l'exposition à la lumière. 50 mg 4,4'-azipentanoate de succinimidyle (sulfo-SDA de la société ThermoScientific) a été ajouté, sous agitation à température (5 °C). le mélange réactionnel a été agité pendant 24h en laissant la température à augmenter à 22°C et gardant le pH entre 6 et 7 par addition d'une solution aqueuse d'hydroxyde de sodium 0,5 M et de solution aqueuse d'acide chlorohydrique 0,5 M.

Le mélange réactionnel a ensuite été introduit dans un tube de dialyse (Spectra/Por Dialysis Membrane MWCO 3500) et dialysé dans 5 litres d'eau osmosé pendant 48 heures, en remplaçant l'eau 8 fois durant cette opération de dialyse.

Le résidu déposé dans le tube de dialyse a été extrait avec de l'eau distillée et lyophilisé pour obtenir un produit solide fibreux de couleur jaune (250 mg).

Le produit a été conservé dans un flacon de couleur ambre à - 20 °C .

L'analyse RMN $_1$H dans l'eau deutériée : 6 % de greffage

**Exemple de synthèse 2 (polymère 2)** : Acide Hyaluronique fonctionnalisé à 11 % par des groupes azide

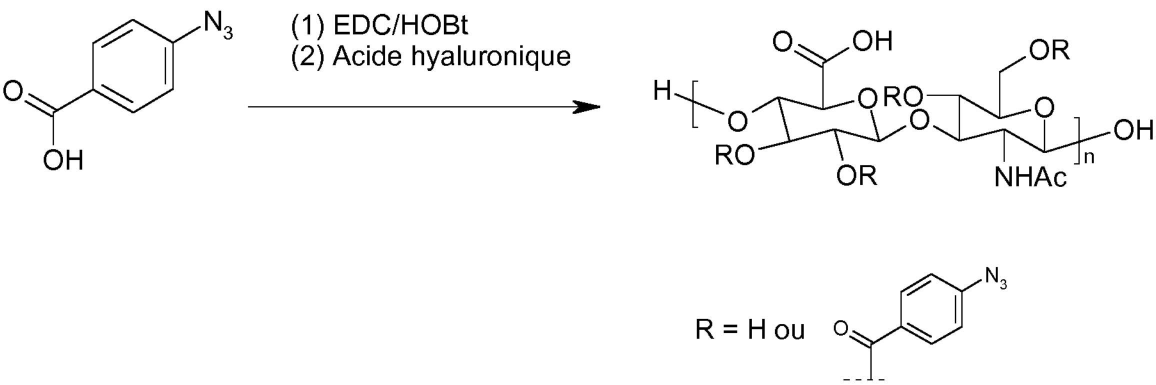


1,63 g (0,01 mole) d'acide 4-azidobenzoique a été mélangé avec 100 ml d'eau distillée dans un ballon en verre

brun. La solution a été agitée rigoureusement puis 1,9 g (0.01 mole) d'hydrochlorure de 1-éthyl-3-(3-diméthylaminopropyl)carbodiimide (EDC) a été ajouté suivi par 68 mg (0,5mmole) de N-hydroxybenzotriazole (HOBt). Le pH a été ajusté à pH 5,2 par addition d'une solution aqueuse d'hydroxyde de sodium 0,1 M. La solution est agitée rigoureusement pendant 50 minutes puis une solution d'acide hyaluronique (Hyacare® 50 de chez Evonik) (2 g dans 70 ml d'eau distillé) a été ajoutée. La solution a été agitée à température ambiante (25 °C) pendant 3 jours.

Le mélange réactionnel a ensuite été introduit dans un tube de dialyse (Spectra/Por Dialysis Membrane MWCO 3500) et dialysé dans 5 litres d'eau pendant 48 heures, en remplaçant l'eau 4 fois durant cette opération de dialyse.
Le résidu déposé dans le tube de dialyse a été extrait avec de l'eau distillée et lyophilisé pour obtenir un produit solide fibreux de couleur jaune.
Ce solide récupéré a été lavé à la température ambiante dans un flacon brun à l'aide d'acétone pendant 2 heures (100 ml par extraction en effectuant 3 extractions).
Le résidu solide a ensuite été filtré pendant 5 minutes puis séché sous vide à la température ambiante pendant 12 heures. On a ainsi obtenu 1,8 g d'un produit solide (poudre) de couleur beige.
Le produit a été conservé dans un vial de couleur ambre à - 20 °C.
L'analyse RMN 1H dans l'eau deutériée : 11 % de greffage

<u>**Exemple de synthèse 3 (polymère 3)**</u> : Acide alginique fonctionnalisé à 10 % par des groupes diazirine

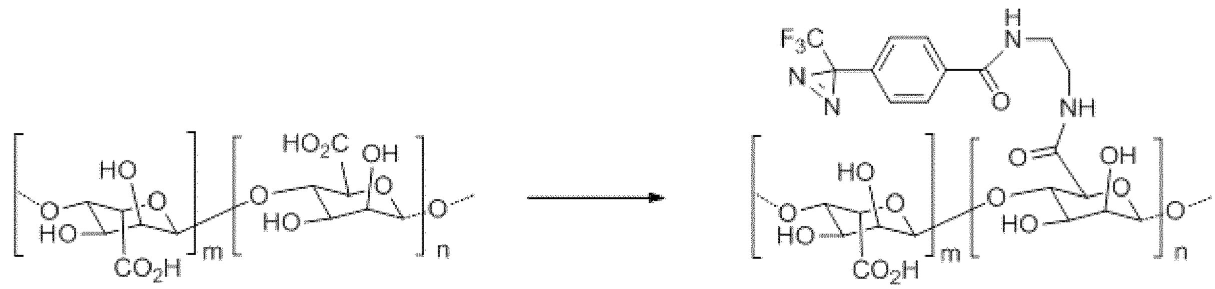

0,65 g (3,68 mmol) d'acide alginique (KELCOSOL de chez ISP) a été mélangé avec 33 ml d'eau distillée dans un ballon en verre brun. Le pH a été ajusté à pH 3,4-3,6 par addition d'une solution aqueuse de l'acide hydrochlorique 0,2 M. La solution a été agitée rigoureusement puis 0,71 g (3,68mmole) d'hydrochlorure de 1-éthyl-3-(3-diméthylaminopropyl)carbodiimide (EDC) dans l'eau (3mL) a été ajouté suivi par 800 mg (3,68 mmole) de N-hydroxysulfosuccinimide. Après 5 minutes d'agitation, N-(2-aminoethyl)-4-(3-(trifluoromethyl)-3H-diazirin-3-yl) benzamide (1.0g) a été ajouté. Le pH a été ajusté à pH 4,7 par addition d'une solution aqueuse de l'acide hydrochlorique 0,2 M. La solution a été agitée à température ambiante (25 °C) pendant 40 heures puis versé dans l'acétone (300mL). Le précipite blanc a été récupéré par filtration. La poudre a été mis dans 40mL d'eau puis introduit dans un tube de dialyse (Spectra/Por Dialysis Membrane MWCO 3500) et dialysé dans 5 litres d'eau pendant 3 jours, en remplaçant l'eau 3 fois durant cette opération de dialyse.
Le produit a été récupéré par lyophilisation pour donner une poudre blanche (600 mg).
Le produit a été conservé dans un vial de couleur ambre à - 20 °C.
L'analyse RMN 1H dans l'eau deutériée : 10 % de greffage

<u>**Exemple 1**</u> : **Mise en évidence de l'effet tenseur des polymères 1 et 2**

On a préparé les compositions suivantes :

Composition 1 : solution aqueuse à 5 % en poids MA d'acide hyaluronique (Hyacare® 50 de chez Evonik)
Composition 2 : solution aqueuse à 5 % en poids MA du polymère 1
Composition 3 : solution aqueuse à 5 % en poids MA du polymère 2
Composition REF : composition aqueuse de Hybridur® 875 polymer dispersion de chez Air Products (dispersion aqueuse à 40 % en poids de particules d'un réseau interpénétré de polymères polyuréthane et acrylique) à 7 % MA.

On a comparé *in vitro* le pouvoir tenseur des polymères 1 et 2 par rapport à un polymère tenseur de référence : Hybridur® 875 polymer dispersion de chez Air Products (dispersion aqueuse à 40 % en poids de particules d'un réseau interpénétré de polymères polyuréthane et acrylique) et également par rapport à de l'acide hyaluronique (Hyacare® 50 de chez Evonik).

L'effet tenseur est mesuré par un test in vitro de rétraction. Ce test consiste à quantifier in vitro le pouvoir tenseur d'un matériau déposé sur un substrat en élastomère (Kimtech nitrile reference 90627 de chez Kimberly Clark) ayant un module de l'ordre de 20 MPa et une épaisseur de 100 $\mu$m.

26 $\mu$l de chaque composition de polymère a été déposée sur une éprouvette rectangulaire (9x40mm) d'élastomère. Certaines des éprouvettes traitées ont été irradiées pendant une minute avec un appareil simulateur de soleil ORIEL de la société ORIEL-LOT.

Après 3 heures de séchage à 22$\pm$3°C et 40$\pm$10% d'humidité relative, l'effet tenseur exercé par le polymère déposé sur l'éprouvette est directement relié à la diminution de la largeur au centre de l'éprouvette. L'effet tenseur (ET1) peut alors se quantifier de la façon suivante:

$$\text{Effet tenseur (ET1) en \%} = (L_0 - L_1 / L_0) \times 100$$

Lo = largeur initiale 9 mm

et L1 = largeur après 3 heures de séchage

On a ensuite évalué la rémanence à la sueur synthétique de l'effet tenseur observé. Les dépôts ont été rincés en projetant sur la bandelette à une distance de 5 cm 10 $\mu$l d'une solution aqueuse 0,9M de NaCl (sueur synthétique).

Les dépôts ont été séchés pendant 3 heures à 22$\pm$3°C et 40$\pm$10% d'humidité relative, puis on a mesuré à nouveau l'effet tenseur après lavage (ET2) en mesurant la largeur L2 de l'éprouvette.

$$\text{Effet tenseur (ET2) en \%} = (L_0 - L2 / L_0) \times 100$$

avec L2 = largeur de l'éprouvette après rinçage et 3 heures de séchage

On a obtenu les résultats suivants :

| Exemple | Compo sition | Polymère | Irradié (oui/non) | Effet tenseur (ET1) (avant lavage) | Effet tenseur (ET2) (après lavage) |
|---|---|---|---|---|---|
| A | 1 | acide hyaluronique | Non | 33% | 11% |
| B | 2 | Polymère 1 | Non | 66 % | 33 % |
| C | 2 | Polymère 1 | oui | 66 % | 66 % |
| D | 3 | Polymère2 | Non | 55 % | 22 % |
| E | 3 | Polymère 2 | oui | 55 % | 55 % |
| F | REF | Hybridur® 875 polymer dispersion | Non | 55% | 22% |

Les résultats obtenus montrent que le polymère de l'exemple 1 ainsi que celui de l'exemple 2 selon l'invention, après irradiation du dépôt (exemples C et E) permettent d'obtenir un bon effet tenseur avant et après lavage. L'effet tenseur obtenu présente donc une bonne rémanence à la sueur.

**Exemple 4** :

On prépare un gel anti-rides ayant la composition suivante :

| | | |
|---|---|---|
| - polymère de l'exemple de synthèse 1 | | 2 g |
| - hydroxyéthyl cellulose (NATROSOL® 250 HHR CS de chez Ashland) | | 0,3 g |
| - Conservateurs | qs | |
| - Eau | qsp | 100 g |

La composition obtenue est appliquée sur le visage puis on irradie la surface de la peau traitée avec une lumière blanche (lampe Lite-Pad de la société Reicorp) pendant 5 minutes. Le traitement appliqué permet de lisser efficacement

les rides.

**Exemple 5 :**

On prépare un gel anti-rides ayant la composition suivante :

| | | |
|---|---|---|
| - polymère de l'exemple 2 | | 1 g |
| - hydroxyéthyl cellulose (NATROSOL® 250 HHR CS de chez Ashland) | | 0,3 g |
| - Conservateurs | qs | |
| - Eau | qsp | 100 g |

La composition obtenue est appliquée sur le visage puis on irradie la surface de la peau traitée avec une lumière blanche (lampe Lite-Pad de la société Reicorp) pendant 15 minutes. Le traitement appliqué permet de lisser efficacement les rides.

**Exemple 6 : Mise en évidence de l'effet tenseur du polymère 3**

On a évalué le pouvoir tenseur du polymère 3 selon le protocole décrit à l'exemple 1
Composition 4 : solution aqueuse à 2 % en poids MA du polymère 3

On a obtenu les résultats suivants :

| Exemple | Compo sition | Polymère | Irradié (oui/non) | Effet tenseur (ET1) (avant lavage) | Effet tenseur (ET2) (après lavage) |
|---|---|---|---|---|---|
| G | 4 | Polymère 3 | Non | 55 % | 22 % |
| H | 4 | Polymère 3 | oui | 55 % | 55 % |
| F | REF | Hybridur® 875 polymer dispersion | Non | 55% | 22% |

Les résultats obtenus montrent que le polymère de l'exemple 3 après irradiation du dépôt (exemple H) permet d'obtenir un bon effet tenseur avant et après lavage. L'effet tenseur obtenu présente donc une bonne rémanence à la sueur.

**Exemple 7** :

On prépare un gel anti-rides ayant la composition suivante :

| | | |
|---|---|---|
| - polymère de l'exemple 3 | | 1 g |
| - hydroxyéthyl cellulose (NATROSOL® 250 HHR CS de chez Ashland) | | 0,3 g |
| - Conservateurs | qs | |
| - Eau | qsp | 100 g |

La composition obtenue est appliquée sur le visage puis on irradie la surface de la peau traitée avec une lumière blanche (lampe Lite-Pad de la société Reicorp) pendant 15 minutes. Le traitement appliqué permet de lisser efficacement les rides.

## Revendications

1. Polymère polysaccharide greffé de groupements photoactifs de type azide ou diazirine de formule (I) :

$$PS\text{-}(O\text{-}CO\text{--}L\text{-}X)_a(OH)_b \qquad (I)$$

dans laquelle PS désigne le squelette de base du polysaccharide portant les groupes hydroxyle ;
L est un groupe divalent hydrocarboné comprenant de 1 à 20 atome de carbone, de préférence de 2 à 10 atomes

de carbone, linéaire, ramifié ou cyclique, saturé ou insaturé, et pouvant être interrompu par un ou plusieurs hétéroatomes non adjacents choisis parmi le soufre, l'oxygène ou groupements -NH-, -COO-,-CONH-, -O-CO-NH-,-NH-CO-NH-, ledtit groupe divalent pouvant éventuellement être substitué par un ou plusieurs groupes choisi parmi les groupes hydroxyle, amine, thiol, acide carboxylique, amide, cyano, acyl $(C_1-C_4)$amino ;
X désigne un groupe photoactif de type azide ou diazirine
a désigne le taux de groupe OH substitué par le groupe photoactif
b désigne le taux de groupe OH libres non substitués
a étant compris entre 0,02 et 0,5 ; b étant compris entre 0,5 et 0,98 ;
et a + b = 1 ;
à l'exception des composés (I) pour lesquels :

PS est le dextran et $-L-X = -(CH_2)_5-N_3$
PS est l'acide hyaluronique et $-L-X = -(CH_2)_3-N_3$

**2.** Polymère selon la revendication précédente, **caractérisé en ce que** le polysaccharide comprend un ou plusieurs motifs de base choisi parmi l'acide uronique, l'acide glucuronique, l'acide manuronique et de préférence l'acide uronique.

**3.** Polymère selon l'une des revendications précédentes, **caractérisé en ce que** le polysaccharide est choisi parmi l'acide hyaluronique, le chondroitine, le chondroitine sulfate, l'acide alginique, l'héparine, l'héparine sulfate, la gomme de xanthane, le dextran, la cellulose.

**4.** Polymère selon l'une des revendications précédentes, **caractérisé en ce que** le polysaccharide est l'acide hyaluronique ou l'acide alginique, et de préférence l'acide hyaluronique.

**5.** Polymère selon l'une des revendications précédentes, **caractérisé en ce que** a est compris entre 0,02 et 0,4 et b est compris entre 0,6 et 0,98 , de préférence, a est compris entre 0,02 et 0,3 et b est compris entre 0,7 et 0,98, préférentiellement a est compris entre 0,04 et 0,2 et b est compris entre 0,8 et 0,96, plus préférentiellement a est compris entre 0,04 et 0,15 ; b est compris entre 0,85 et 0,96.

**6.** Polymère selon l'une des revendications précédentes, **caractérisé en ce que** L est choisi parmi les groupes suivants :

| | | |
|---|---|---|
| 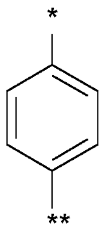 | 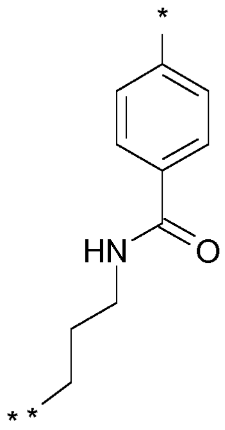 | 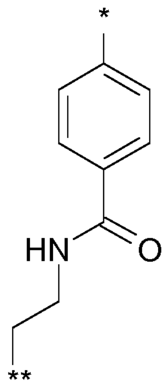 |
| 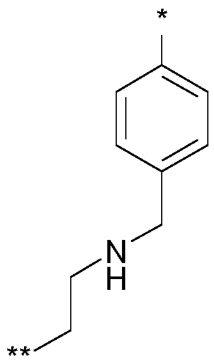 | $*-CH_2-[CH_2]_m-**$<br>m = 1-3 | 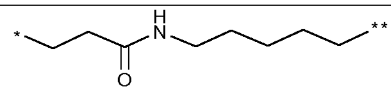 |

*représentant la liaison avec le groupement photoactif X

** représentant la liaison avec le groupe ester lié au polymère PS (groupe PS-O-CO-)

**7.** Polymère selon l'une des revendications précédentes, **caractérisé en ce que** le groupe photoactif X peut être choisi parmi les groupes suivants :

| | | | |
|---|---|---|---|
| $*-N_3$ | 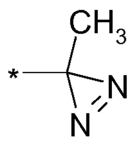 | 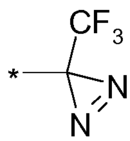 | 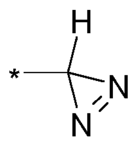 |

**8.** Polymère selon l'une des revendications précédentes, **caractérisé en ce que** le groupement X-L- est choisi parmi :

| | | |
|---|---|---|
| 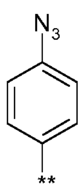<br>(i) | 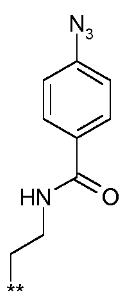<br>(ii) | 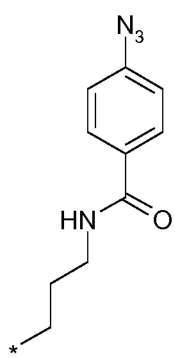<br>(iii) |

(suite)

| | | |
|---|---|---|
| $N_3$-$CH_2$-$CH_2$-** (iv) | 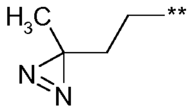 (v) | 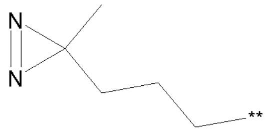 (vi) |
| 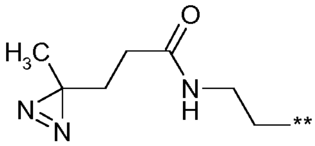 (vii) | 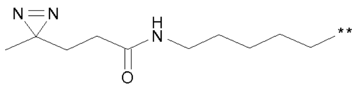 (viii) | 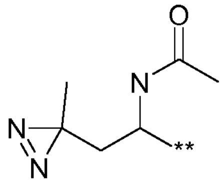 (ix) |
| 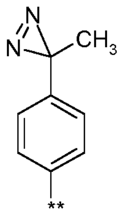 (x) | 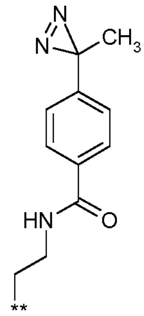 (xi) | 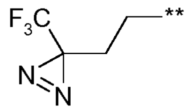 (xii) |
| 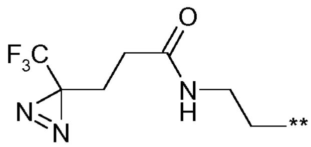 (xiii) | 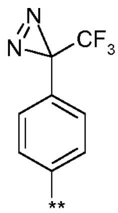 (xiv) | 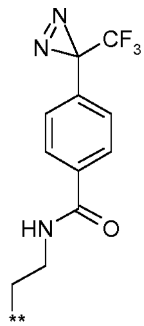 (xv) |
| ** représentant la liaison avec le groupe ester lié au polymere PS (groupe -PS-O-CO-) | | |

et de préférence choisi parmi :

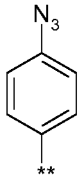

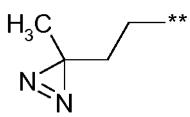

**9.** Polymère selon l'une quelconque des revendications précédentes, **caractérisé en ce qu'**il a un poids moléculaire moyen en poids allant de 5000 à 1 000 000 daltons, de préférence allant de 10000 à 500 000 daltons, préférentiellement allant de 15000 à 350 000 daltons.

**10.** Composition comprenant, dans un milieu physiologiquement acceptable un polysaccharide greffé (I) selon l'une des revendications précédentes.

**11.** Composition selon la revendication précédente, **caractérisée en ce que** le polysaccharide greffé (I) est présent en une teneur allant de 0,1 à 10 % en poids, par rapport au poids total de la composition, de préférence allant de 0,5 % à 10 % en poids, et préférentiellement allant de 1 à 8 % en poids, et plus préférentiellement allant de 1 % à 6 % en poids.

**12.** Composition selon la revendication 10 ou 11 , **caractérisée en ce qu'**elle comprend un adjuvant cosmétique choisi parmi l'eau, les émulsionnants, les conservateurs, les séquestrants, les parfums, les épaississants, les huiles, les cires, les polymères filmogènes.

**13.** Composition selon l'une des revendications 10 à 12, **caractérisée en ce qu'**elle se présente sous forme d'une émulsion H/E ou d'un gel aqueux.

**14.** Procédé cosmétique de soin de la peau, plus particulièrement de la peau du visage, en particulier de la peau ridée, comprenant :

(i) une étape consistant à appliquer sur la peau une composition comprenant, dans un milieu physiologiquement acceptable, un polysaccharide greffé (I),
(ii) une étape consistant à exposer la peau traitée à un rayonnement lumineux, de préférence pendant au moins 5 secondes,
le polymère greffé (I) étant un polymère polysaccharide greffé de groupements photoactifs de type azide ou diazirine de formule :

$$PS\text{-}(O\text{-}CO\text{--}L\text{-}X)_a(OH)_b \qquad (I)$$

dans laquelle PS désigne le squelette de base du polysaccharide portant les groupes hydroxyle ;
L est un groupe divalent hydrocarboné comprenant de 1 à 20 atome de carbone, de préférence de 2 à 10 atomes de carbone, linéaire, ramifié ou cyclique, saturé ou insaturé, et pouvant être interrompu par un ou plusieurs hétéroatomes non adjacents choisis parmi le soufre, l'oxygène ou groupements -NH-, -COO-,-CONH-, -O-CO-NH-,-NH-CO-NH-, ledtit groupe divalent pouvant éventuellement être substitué par un ou plusieurs groupes choisi parmi les groupes hydroxyle, amine, thiol, acide carboxylique, amide, cyano, acyl ($C_1$-$C_4$)amino ;
X désigne un groupe photoactif de type azide ou diazirine
a désigne le taux de groupe OH substitué par le groupe photoactif
b désigne le taux de groupe OH libres non substitués
a étant compris entre 0,02 et 0,5 ; b étant compris entre 0,5 et 0,98 ;
et a + b = 1 ;

**15.** Procédé selon la revendication précédente, **caractérisé en ce que** le polysaccharide comprend un ou plusieurs motifs de base choisi parmi l'acide uronique, l'acide glucuronique, l'acide manuronique et de préférence l'acide uronique.

**16.** Procédé selon l'une des revendications 14 ou 15, **caractérisé en ce que** le polysaccharide est choisi parmi l'acide hyaluronique, le chondroitine, le chondroitine sulfate, l'acide alginique, l'héparine, l'héparine sulfate, la gomme de xanthane, le dextran, la cellulose.

**17.** Procédé selon l'une des revendications 14 à 16, **caractérisé en ce que** le polysaccharide est l'acide hyaluronique ou l'acide alginique, et de préférence l'acide hyaluronique.

**18.** Procédé selon l'une des revendications 14 à 17, **caractérisé en ce que** a est compris entre 0,02 et 0,4 et b est compris entre 0,6 et 0,98 , de préférence, a est compris entre 0,02 et 0,3 et b est compris entre 0,7 et 0,98, préférentiellement a est compris entre 0,04 et 0,2 et b est compris entre 0,8 et 0,96, plus préférentiellement a est compris entre 0,04 et 0,15 ; b est compris entre 0,85 et 0,96.

**19.** Procédé selon l'une des revendications précédentes, **caractérisé en ce que** L est choisi parmi les groupes suivants :

| | | |
|---|---|---|
| 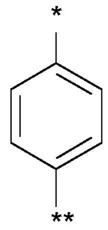 | 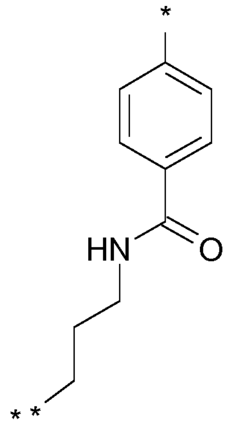 | 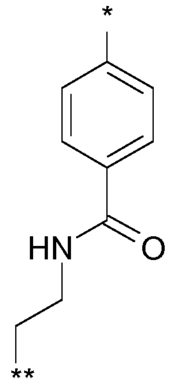 |
| 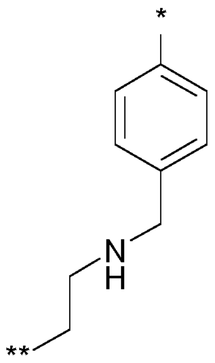 | $*-CH_2-[CH_2]_m-**$<br>m = 1-3 | 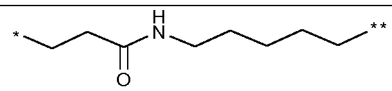 |

*représentant la liaison avec le groupement photoactif X

** représentant la liaison avec le groupe ester lié au polymère PS (groupe PS-O-CO-)

**20.** Procédé selon l'une des revendications 14 à 19, **caractérisé en ce que** le groupe photoactif X peut être choisi parmi les groupes suivants :

| | | | |
|---|---|---|---|
| $*-N_3$ | 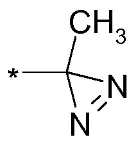 | 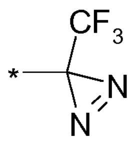 | 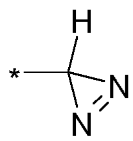 |

**21.** Procédé selon l'une des revendications 14 à 20, **caractérisé en ce que** le groupement X-L- est choisi parmi :

| | | |
|---|---|---|
| 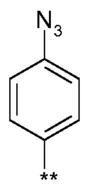<br>(i) | 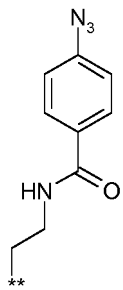<br>(ii) | 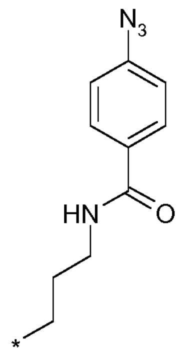<br>(iii) |

(suite)

| | | |
|---|---|---|
| $N_3$-$CH_2$-$CH_2$-** (iv) | 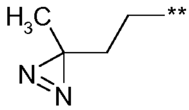 (v) | 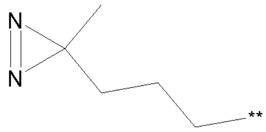 (vi) |
| 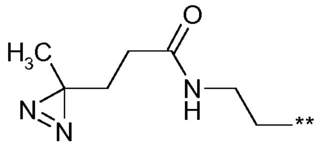 (vii) | 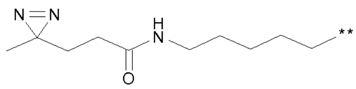 (viii) | 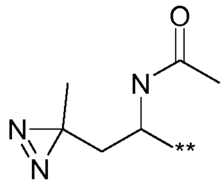 (ix) |
| 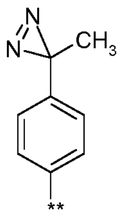 (x) | 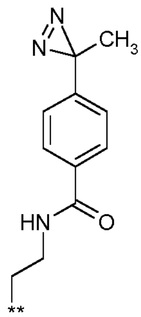 (xi) | 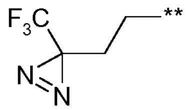 (xii) |
| 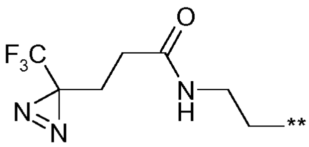 (xiii) | 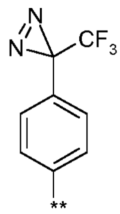 (xiv) | 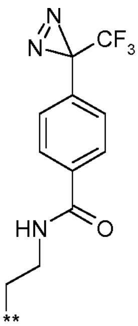 (xv) |
| ** représentant la liaison avec le groupe ester lié au polymere PS (groupe -PS-O-CO-) | | |

et de préférence choisi parmi :

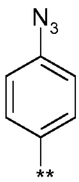

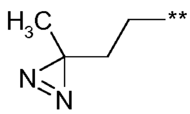

**22.** Procédé selon l'une quelconque des revendications 14 à 21, **caractérisé en ce qu'**il a un poids moléculaire moyen en poids allant de 5000 à 1 000 000 daltons, de préférence allant de 10000 à 500 000 daltons, préférentiellement allant de 15000 à 350 000 daltons.

**23.** Procédé selon l'une quelconque des revendications 14 à 21, **caractérisé en ce que** le polysaccharide greffé (I) est présent en une teneur allant de 0,1 à 10 % en poids, par rapport au poids total de la composition, de préférence

allant de 0,5 % à 10 % en poids, et préférentiellement allant de 1 à 8 % en poids, et plus préférentiellement allant de 1 % à 6 % en poids.

**24.** Procédé selon l'une quelconque des revendications 14 à 23, dans lequel l'étape consistant à appliquer un rayonnement lumineux est effectuée après ou en même temps (simultanément) que l'étape consistant à appliquer la composition cosmétique comprenant le polysaccharide greffé (I).

**25.** Procédé selon l'une quelconque des revendications 14 à 23, dans lequel l'étape (ii) consistant à appliquer un rayonnement lumineux est effectuée après l'étape (i) consistant à appliquer la composition cosmétique comprenant le polysaccharide greffé (I).

**26.** Procédé selon l'une quelconque des revendications 14 à 25, **caractérisé en ce que** le rayonnement lumineux est la lumière naturelle ou à la lumière artificielle de longueur d'onde comprise entre 360 et 600 nm.

**27.** Procédé selon l'une des revendications 14 à 25, dans lequel le rayonnement lumineux a une source choisie parmi les lampes d'arc telles que les lampes à xénon et les lampes à mercure; les lampes à fluorescence; les lampes à incandescence telles que les halogènes, les LEDs et les lasers.

**28.** Procédé selon l'une des revendication 14 à 27, dans lequel la durée d'exposition au rayonnement lumineux est d'au moins 5 secondes, de préférence cette durée d'exposition peut aller de 10 secondes à 15 minutes, notamment entre 15 secondes et 10 minutes, encore mieux entre 20 secondes et 5 minutes.

**29.** Procédé selon l'une quelconque des revendications 14 à 28, **caractérisé en ce qu'**il est destiné à atténuer les rides.

## Patentansprüche

**1.** Mit photoaktiven Gruppen vom Azid- oder Diazirin-Typ gepfropftes Polysaccharid-Polymer der Formel (I) :

$$PS\text{-}(O\text{-}CO\text{--}L\text{-}X)_a(OH)_b \qquad (I)$$

wobei PS für das Grundgerüst des Polysaccharids, das die Hydroxylgruppen trägt, steht;
L eine lineare, verzweigte oder cyclische, gesättigte oder ungesättigte zweiwertige Kohlenwasserstoffgruppe, die 1 bis 20 Kohlenstoffatome, vorzugsweise 2 bis 10 Kohlenstoffatome, aufweist und durch ein oder mehrere nicht benachbarte Heteroatome, die aus Schwefel, Sauerstoff oder -NH-, -COO-, -CONH-, -O-CO-NH- oder -NH-CO-NH-Gruppen ausgewählt sind, substituiert sein kann, ist, wobei die zweiwertige Gruppe gegebenenfalls durch eine oder mehrere Gruppen, die aus Hydroxyl-, Amin-, Thiol-, Carbonsäure-, Amid-, Cyano- und Acyl ($C_1$-$C_4$) aminogruppen ausgewählt sind, substituiert sein kann;
X für eine photoaktive Gruppe von Azid- oder Diazirin-Typ steht;
a für den Gehalt an OH-Gruppen, die durch die photoaktive Gruppe substituiert sind, steht;
b für den Gehalt an unsubstituierten freien OH-Gruppen steht;
wobei a zwischen 0,02 und 0,5 liegt und b zwischen 0,5 und 0,98 liegt;
und a + b = 1; mit Ausnahme von Verbindungen der Formel (I), für die:

PS Dextran ist und -L-X = $-(CH_2)_5-N_3$,
PS Hyaluronsäure ist und -L-X = $-(CH_2)_3-N_3$.

**2.** Polymer nach dem vorhergehenden Anspruch, **dadurch gekennzeichnet, dass** das Polysaccharid eine oder mehrere Grundeinheiten umfasst, die aus Uronsäure, Glucoronsäure, Mannuronsäure und vorzugsweise Uronsäure ausgewählt sind.

**3.** Polymer nach einem der vorhergehenden Ansprüche, **dadurch gekennzeichnet, dass** das Polysaccharid aus Hyaluronsäure, Chondroitin, Chondroitinsulfat, Alginsäure, Heparin, Heparinsulfat, Xanthangummi, Dextran und Cellulose ausgewählt ist.

**4.** Polymer nach einem der vorhergehenden Ansprüche, **dadurch gekennzeichnet, dass** es sich bei dem Polysaccharid um Hyaluronsäure oder Alginsäure, vorzugsweise Hyaluronsäure, handelt.

5. Polymer nach einem der vorhergehenden Ansprüche, **dadurch gekennzeichnet, dass** a zwischen 0,02 und 0,4 liegt und b zwischen 0,6 und 0,98 liegt, vorzugsweise a zwischen 0,02 und 0,3 liegt und b zwischen 0,7 und 0,98 liegt, bevorzugt a zwischen 0,04 und 0,2 liegt und b zwischen 0,8 und 0,96 liegt, weiter bevorzugt a zwischen 0,04 und 0,15 liegt und b zwischen 0,85 und 0,96 liegt.

6. Polymer nach einem der vorhergehenden Ansprüche, **dadurch gekennzeichnet, dass** L aus den folgenden Gruppen ausgewählt ist:

| | | |
|---|---|---|
| 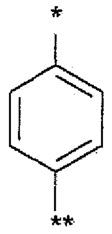 | 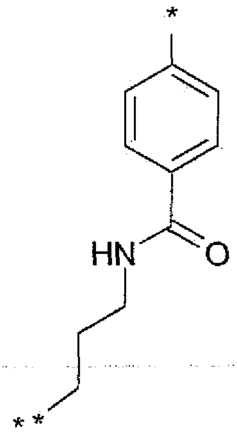 | 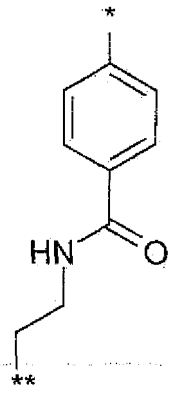 |
| 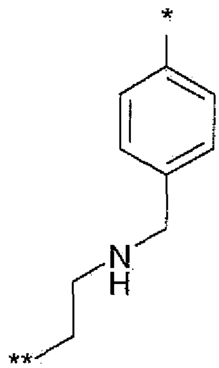 | $*-CH_2-[CH_2]_m-**$<br>m = 1-3 | 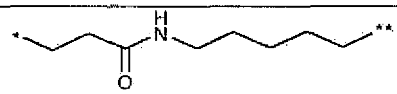 |

wobei * für die Bindung mit der photoaktiven Gruppe X steht,
wobei ** für die Bindung mit der an das Polymer PS gebundenen Estergruppe (PS-O-CO-Gruppe) steht.

7. Polymer nach einem der vorhergehenden Ansprüche, **dadurch gekennzeichnet, dass** die photoaktive Gruppe X aus den folgenden Gruppen ausgewählt sein kann:

| | | | |
|---|---|---|---|
| $*-N_3$ | 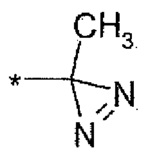 | 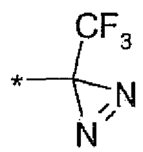 | 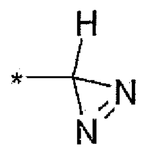 |

8. Polymer nach einem der vorhergehenden Ansprüche, **dadurch gekennzeichnet, dass** die Gruppe X-L- aus:

| | | |
|---|---|---|
| 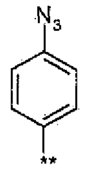 (i) | 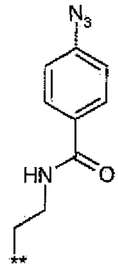 (ii) | 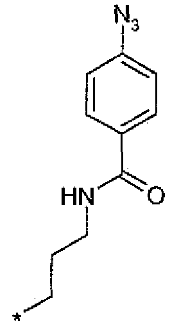 (iii) |
| $N_3$-$CH_2$-$CH_2$-∗∗ (iv) | 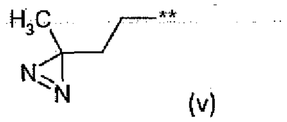 (v) | 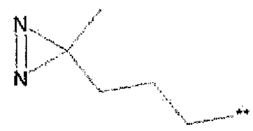 (vi) |
| 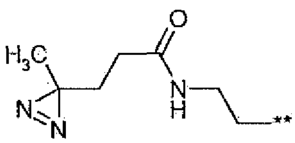 (vii) | 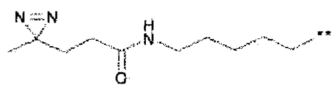 (viii) | 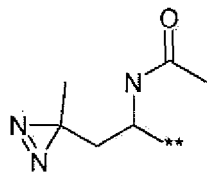 (ix) |
| 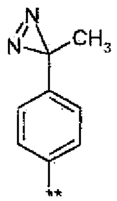 (x) | 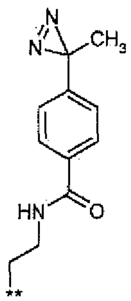 (xi) | 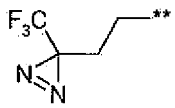 (xii) |
| | | |
| 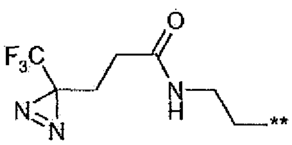 (xiii) | 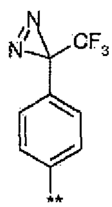 (xiv) | 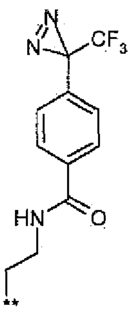 (xv) |
| wobei ** für die Bindung mit der an das Polymer PS gebundenen Estergruppe (PS-O-CO-Gruppe) steht, | | |

ausgewählt ist und vorzugsweise aus:

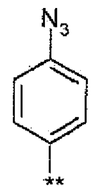

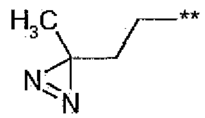

ausgewählt ist.

9. Polymer nach einem der vorhergehenden Ansprüche, **dadurch gekennzeichnet, dass** es ein gewichtsmittleres Molekulargewicht im Bereich von 5000 bis 1.000.000 Dalton, vorzugsweise im Bereich von 10.000 bis 500.000 Dalton, bevorzugt im Bereich von 15.000 bis 350.000 Dalton, aufweist.

10. Zusammensetzung, die in einem physiologisch unbedenklichen Medium ein gepfropftes Polysaccharid (I) nach einem der vorhergehenden Ansprüche umfasst.

11. Zusammensetzung nach dem vorhergehenden Anspruch, **dadurch gekennzeichnet, dass** das gepfropfte Polysaccharid (I) in einem Gehalt im Bereich von 0,1 bis 10 Gew.-%, bezogen auf das Gesamtgewicht der Zusammensetzung, vorzugsweise im Bereich von 0,5 bis 10 Gew.-%, bevorzugt im Bereich von 1 bis 8 Gew.-% und weiter bevorzugt im Bereich von 1 bis 6 Gew.-% vorliegt.

12. Zusammensetzung nach Anspruch 10 oder 11, **dadurch gekennzeichnet, dass** sie einen kosmetischen Hilfsstoff umfasst, der aus Wasser, Emulgatoren, Konservierungsmitteln, Sequestriermitteln, Duftstoffen, Verdickern, Ölen, Wachsen und filmbildenden Polymeren ausgewählt ist.

13. Zusammensetzung nach einem der Ansprüche 10 bis 12, **dadurch gekennzeichnet, dass** sie in Form einer O/W-Emulsion oder eines wässrigen Gels vorliegt.

14. Kosmetisches Verfahren zur Pflege der Haut, spezieller der Gesichtshaut, insbesondere von faltiger Haut, umfassend:

(i) einen Schritt, der darin besteht, dass man auf die Haut eine Zusammensetzung aufbringt, die in einem physiologisch unbedenklichen Medium ein gepfropftes Polysaccharid (I) umfasst,
(ii) einen Schritt, der darin besteht, dass man auf die Haut Lichtstrahlung einwirken lässt, vorzugsweise über einen Zeitraum von mindestens 5 Sekunden,
wobei es sich bei dem gepfropften Polymer (I) um ein mit photoaktiven Gruppen vom Azid- oder Diazirin-Typ gepfropftes Polysaccharid-Polymer der Formel (I) handelt:

$$PS\text{-}(O\text{-}CO\text{--}L\text{-}X)_a(OR)_b \qquad (I)$$

wobei PS für das Grundgerüst des Polysaccharids, das die Hydroxylgruppen trägt, steht;
L eine lineare, verzweigte oder cyclische, gesättigte oder ungesättigte zweiwertige Kohlenwasserstoffgruppe, die 1 bis 20 Kohlenstoffatome, vorzugsweise 2 bis 10 Kohlenstoffatome, aufweist und durch ein oder mehrere nicht benachbarte Heteroatome, die aus Schwefel, Sauerstoff oder -NH-, -COO-, -CONH-, -O-CO-NH- oder -NH-CO-NH-Gruppen ausgewählt sind, substituiert sein kann, ist, wobei die zweiwertige Gruppe gegebenenfalls durch eine oder mehrere Gruppen, die aus Hydroxyl-, Amin-, Thiol-, Carbonsäure-, Amid-, Cyano- und Acyl ($C_1$-$C_4$) aminogruppen ausgewählt sind, substituiert sein kann;
X für eine photoaktive Gruppe vom Azid- oder Diazirin-Typ steht;
a für den Gehalt an OH-Gruppen, die durch die photoaktive Gruppe substituiert sind, steht;
b für den Gehalt an unsubstituierten freien OH-Gruppen steht;
wobei a zwischen 0,02 und 0,5 liegt und b zwischen 0,5 und 0,98 liegt;
und a + b = 1.

15. Verfahren nach dem vorhergehenden Anspruch, **dadurch gekennzeichnet, dass** das Polysaccharid eine oder mehrere Grundeinheiten umfasst, die aus Uronsäure, Glucoronsäure, Mannuronsäure und vorzugsweise Uronsäure ausgewählt sind.

**16.** Verfahren nach einem der Ansprüche 14 oder 15, **dadurch gekennzeichnet, dass** das Polysaccharid aus Hyaluronsäure, Chondroitin, Chondroitinsulfat, Alginsäure, Heparin, Heparinsulfat, Xanthangummi, Dextran und Cellulose ausgewählt ist.

**17.** Verfahren nach einem der Ansprüche 14 bis 16, **dadurch gekennzeichnet, dass** es sich bei dem Polysaccharid um Hyaluronsäure oder Alginsäure, vorzugsweise Hyaluronsäure, handelt.

**18.** Verfahren nach einem der Ansprüche 14 bis 17, **dadurch gekennzeichnet, dass** a zwischen 0,02 und 0,4 liegt und b zwischen 0,6 und 0,98 liegt, vorzugsweise a zwischen 0,02 und 0,3 liegt und b zwischen 0,7 und 0,98 liegt, bevorzugt a zwischen 0,04 und 0,2 liegt und b zwischen 0,8 und 0,96 liegt, weiter bevorzugt a zwischen 0,04 und 0,15 liegt und b zwischen 0,85 und 0,96 liegt.

**19.** Verfahren nach einem der vorhergehenden Ansprüche, **dadurch gekennzeichnet, dass** L aus den folgenden Gruppen ausgewählt ist:

| | | |
|---|---|---|
| 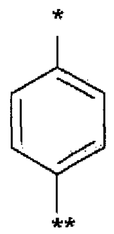 | 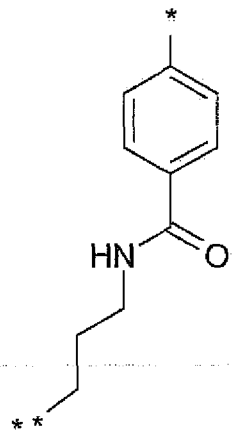 | 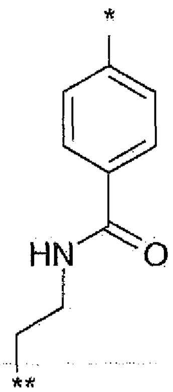 |
| 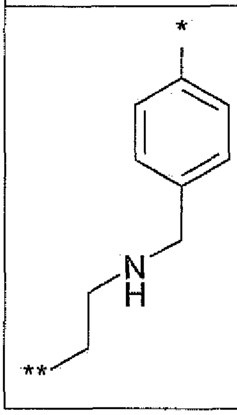 | $*-CH_2-[CH_2]_m-**$<br>m = 1-3 | 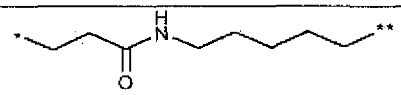 |

wobei * für die Bindung mit der photoaktiven Gruppe X steht,
wobei ** für die Bindung mit der an das Polymer PS gebundenen Estergruppe (PS-O-CO-Gruppe) steht.

**20.** Verfahren nach einem der Ansprüche 14 bis 19, **dadurch gekennzeichnet, dass** die photoaktive Gruppe X aus den folgenden Gruppen ausgewählt sein kann:

| | | | |
|---|---|---|---|
| $*-N_3$ | 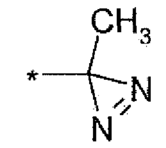 | 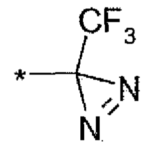 | 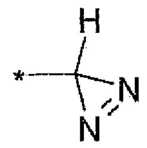 |

**21.** Verfahren nach einem der Ansprüche 14 bis 20, **dadurch gekennzeichnet, dass** die Gruppe X-L- aus:

| | | |
|---|---|---|
| 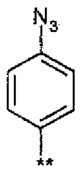 (i) | 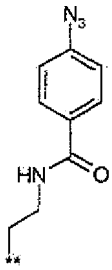 (ii) | 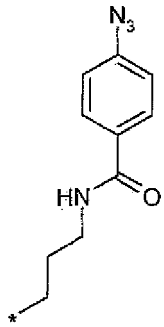 (iii) |
| $N_3$-$CH_2$-$CH_2$-** (iv) | 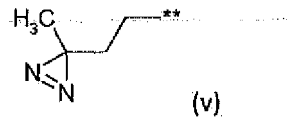 (v) | 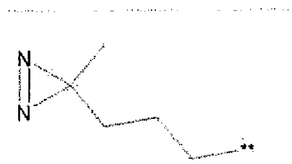 (vi) |
| 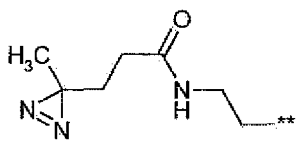 (vii) | 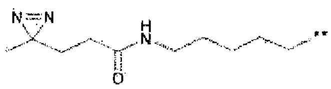 (viii) | 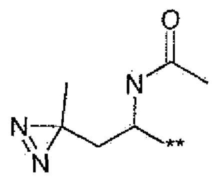 (ix) |
| 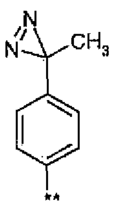 (x) | 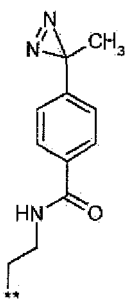 (xi) | 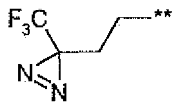 (xii) |
| | | |

(fortgesetzt)

| | | |
|---|---|---|
| 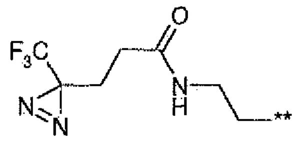 (xiii) | 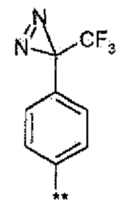 (xiv) | 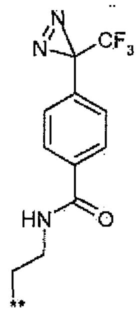 (xv) |
| wobei ** für die Bindung mit der an das Polymer PS gebundenen Estergruppe (PS-O-CO-Gruppe) steht, | | |

ausgewählt ist und vorzugsweise aus:

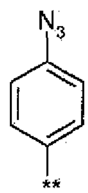

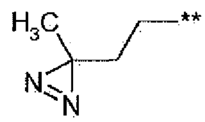

ausgewählt ist.

22. Verfahren nach einem der Ansprüche 14 bis 21, **dadurch gekennzeichnet, dass** es ein gewichtsmittleres Molekulargewicht im Bereich von 5000 bis 1.000.000 Dalton, vorzugsweise im Bereich von 10.000 bis 500.000 Dalton, bevorzugt im Bereich von 15.000 bis 350.000 Dalton, aufweist.

23. Verfahren nach einem der Ansprüche 14 bis 21, **dadurch gekennzeichnet, dass** das gepfropfte Polysaccharid (I) in einem Gehalt im Bereich von 0,1 bis 10 Gew.-%, bezogen auf das Gesamtgewicht der Zusammensetzung, vorzugsweise im Bereich von 0,5 bis 10 Gew.-%, bevorzugt im Bereich von 1 bis 8 Gew.-% und weiter bevorzugt im Bereich von 1 bis 6 Gew.-% vorliegt.

24. Verfahren nach einem der Ansprüche 14 bis 23, bei dem der Schritt, der darin besteht, dass man eine Lichtstrahlung aufbringt, nach dem Schritt, der darin besteht, dass man die kosmetische Zusammensetzung, die das gepfropfte Polysaccharid (I) umfasst, oder zur gleichen Zeit wie dieser Schritt (gleichzeitig) durchgeführt wird.

25. Verfahren nach einem der Ansprüche 14 bis 23, bei dem der Schritt (ii), der darin besteht, dass man eine Lichtstrahlung aufbringt, nach dem Schritt (i), der darin besteht, dass man die kosmetische Zusammensetzung, die das gepfropfte Polysaccharid (I) umfasst, durchgeführt wird.

26. Verfahren nach einem der Ansprüche 14 bis 25, **dadurch gekennzeichnet, dass** es sich bei der Lichtstrahlung um natürliches Licht oder künstliches Licht mit einer Wellenlänge zwischen 360 und 600 nm handelt.

27. Verfahren nach einem der Ansprüche 14 bis 25, wobei die Lichtstrahlung eine aus Lichtbogenlampen wie Xenonlampen und Quecksilberlampen, Fluoreszenzlampen, Glühlampen wie Halogenlampen, LEDs und Lasern ausgewählte Quelle hat.

28. Verfahren nach einem der Ansprüche 14 bis 27, wobei die Zeit des Einwirkenlassens der Lichtstrahlung mindestens 5 Sekunden beträgt und diese Einwirkungszeit vorzugsweise im Bereich von 10 Sekunden bis 15 Minuten, insbesondere zwischen 15 Sekunden und 10 Minuten und noch besser zwischen 20 Sekunden und 5 Minuten liegen kann.

29. Verfahren nach einem der Ansprüche 14 bis 28, **dadurch gekennzeichnet, dass** es zur Abschwächung von Falten vorgesehen ist.

## Claims

1. Polysaccharide polymer grafted with photoactive groups of azide or diazirine type of formula (I):

$$PS\text{-}(O\text{-}CO\text{--}L\text{-}X)_a(OH)_b \quad (I)$$

in which PS denotes the basic backbone of the polysaccharide bearing the hydroxyl groups;
L is a linear, branched or cyclic, saturated or unsaturated divalent hydrocarbon-based group comprising from 1 to 20 carbon atoms, preferably from 2 to 10 carbon atoms, which may be interrupted with one or more non-adjacent heteroatoms chosen from sulfur, oxygen, or -NH-, -COO-, -CONH-, -O-CO-NH- or -NH-CO-NH- groups, said divalent group being optionally able to be substituted with one or more groups chosen from hydroxyl, amine, thiol, carboxylic acid, amide, cyano, and acyl ($C_1$-$C_4$)amino groups;
X denotes a photoactive group of azide or diazirine type; a denotes the content of OH groups substituted with the photoactive group;
b denotes the content of unsubstituted free OH groups;
a being between 0.02 and 0.5; b being between 0.5 and 0.98;
and a + b = 1;
with the exception of compounds (I) for which:

PS is dextran and -L-X = $-(CH_2)_5-N_3$
PS is hyaluronic acid and -L-X = $-(CH_2)_3-N_3$.

2. Polymer according to the preceding claim, **characterized in that** the polysaccharide comprises one or more base units chosen from uronic acid, glucuronic acid and mannuronic acid, preferably uronic acid.

3. Polymer according to either of the preceding claims, **characterized in that** the polysaccharide is chosen from hyaluronic acid, chondroitin, chondroitin sulfate, alginic acid, heparin, heparin sulfate, xanthan gum, dextran and cellulose.

4. Polymer according to one of the preceding claims, **characterized in that** the polysaccharide is hyaluronic acid or alginic acid, preferably hyaluronic acid.

5. Polymer according to one of the preceding claims, **characterized in that** a is between 0.02 and 0.4 and b is between 0.6 and 0.98, preferably, a is between 0.02 and 0.3 and b is between 0.7 and 0.98, preferentially, a is between 0.04 and 0.2 and b is between 0.8 and 0.96, more preferentially, a is between 0.04 and 0.15; b is between 0.85 and 0.96.

6. Polymer according to one of the preceding claims, **characterized in that** L is chosen from the following groups:

| | | |
|---|---|---|
| 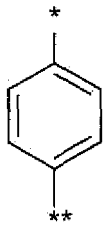 | 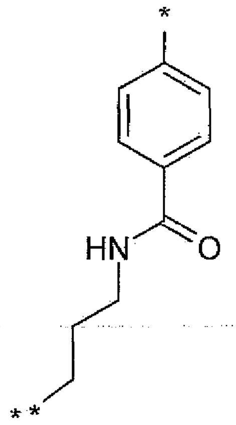 | 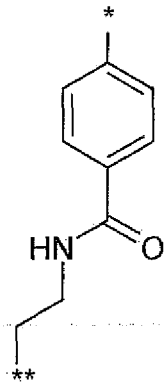 |
| 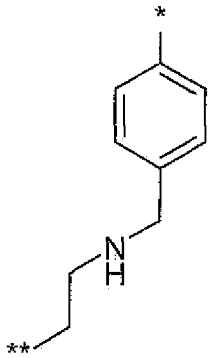 | $*-CH_2-[CH_2]_m-**$<br>m = 1-3 | 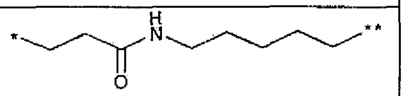 |

* representing the bond with the photoactive group X

** representing the bond with the ester group bonded to the polymer PS (PS-O-CO- group).

7. Polymer according to one of the preceding claims, **characterized in that** the photoactive group X may be chosen from the following groups:

| | | | |
|---|---|---|---|
| $*-N_3$ | 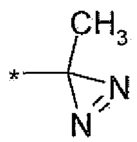 | 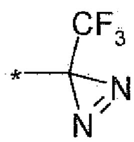 | 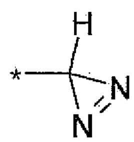 |

8. Polymer according to one of the preceding claims, **characterized in that** the group X-L- is chosen from:

| | | |
|---|---|---|
| 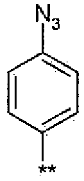 (i) | 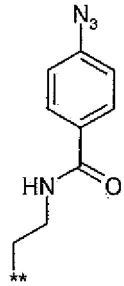 (ii) | 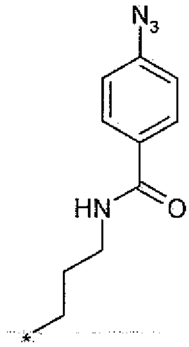 (iii) |
| $N_3$-$CH_2$-$CH_2$-** (iv) | 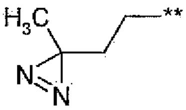 (v) | 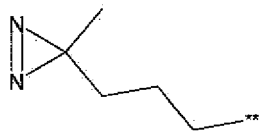 (vi) |
| 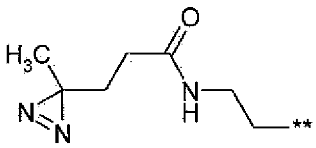 (vii) | 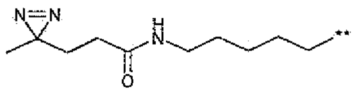 (viii) | 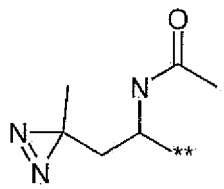 (ix) |
| 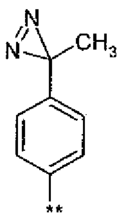 (x) | 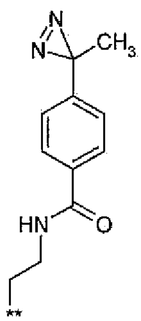 (xi) | 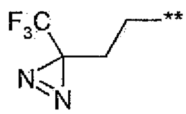 (xii) |
| 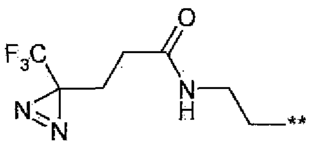 (xiii) | 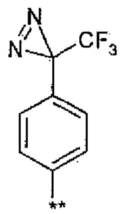 (xiv) | 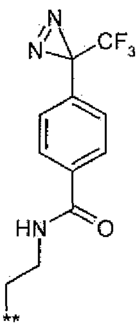 (xv) |
| ** representing the bond with the ester group bonded to the polymer PS (PS-O-CO- group) | | |

and preferably chosen from:

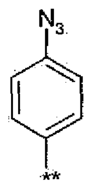

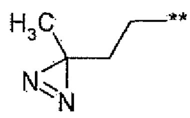

**9.** Polymer according to any one of the preceding claims, **characterized in that** it has a weight-average molecular weight ranging from 5000 to 1 000 000 daltons, preferably ranging from 10 000 to 500 000 daltons and preferentially ranging from 15 000 to 350 000 daltons.

**10.** Composition comprising, in a physiologically acceptable medium, a grafted polysaccharide (I) according to one of the preceding claims.

**11.** Composition according to the preceding claim, **characterized in that** the grafted polysaccharide (I) is present in a content ranging from 0.1% to 10% by weight, relative to the total weight of the composition, preferably ranging from 0.5% to 10% by weight, preferentially ranging from 1% to 8% by weight, and more preferentially ranging from 1% to 6% by weight.

**12.** Composition according to Claim 10 or 11, **characterized in that** it comprises a cosmetic adjuvant chosen from water, emulsifiers, preserving agents, sequestrants, fragrances, thickeners, oils, waxes and film-forming polymers.

**13.** Composition according to one of Claims 10 to 12, **characterized in that** it is in the form of an O/W emulsion or an aqueous gel.

**14.** Cosmetic process for caring for the skin, more particularly facial skin, in particular wrinkled skin, comprising:

(i) a step consisting in applying to the skin a composition comprising, in a physiologically acceptable medium, a grafted polysaccharide (I),
(ii) a step consisting in exposing the treated skin to light radiation, preferably for at least 5 seconds,
the grafted polymer (I) being a polysaccharide polymer grafted with photoactive groups of azide or diazirine type of formula:

$$PS\text{-}(O\text{-}CO\text{--}L\text{-}X)_a(OH)_b \qquad (I)$$

in which PS denotes the basic backbone of the polysaccharide bearing the hydroxyl groups;
L is a linear, branched or cyclic, saturated or unsaturated divalent hydrocarbon-based group comprising from 1 to 20 carbon atoms, preferably from 2 to 10 carbon atoms, which may be interrupted with one or more non-adjacent heteroatoms chosen from sulfur, oxygen, or -NH-, -COO-, -CONH-, -O-CO-NH- or -NH-CO-NH- groups, said divalent group being optionally able to be substituted with one or more groups chosen from hydroxyl, amine, thiol, carboxylic acid, amide, cyano, and acyl ($C_1$-$C_4$)amino groups;
X denotes a photoactive group of azide or diazirine type; a denotes the content of OH groups substituted with the photoactive group;
b denotes the content of unsubstituted free OH groups;
a being between 0.02 and 0.5; b being between 0.5 and 0.98;
and a + b = 1.

**15.** Process according to the preceding claim, **characterized in that** the polysaccharide comprises one or more base units chosen from uronic acid, glucuronic acid and mannuronic acid, preferably uronic acid.

**16.** Process according to either of Claims 14 and 15, **characterized in that** the polysaccharide is chosen from hyaluronic acid, chondroitin, chondroitin sulfate, alginic acid, heparin, heparin sulfate, xanthan gum, dextran and cellulose.

**17.** Process according to one of Claims 14 to 16, **characterized in that** the polysaccharide is hyaluronic acid or alginic acid, preferably hyaluronic acid.

**18.** Process according to one of Claims 14 to 17, **characterized in that** a is between 0.02 and 0.4 and b is between

0.6 and 0.98, preferably, a is between 0.02 and 0.3 and b is between 0.7 and 0.98, preferentially, a is between 0.04 and 0.2 and b is between 0.8 and 0.96, more preferentially, a is between 0.04 and 0.15; b is between 0.85 and 0.96.

**19.** Process according to one of the preceding claims, **characterized in that** L is chosen from the following groups:

| | | |
|---|---|---|
| 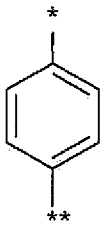 | 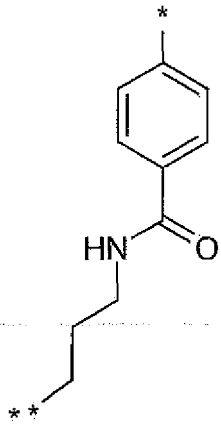 | 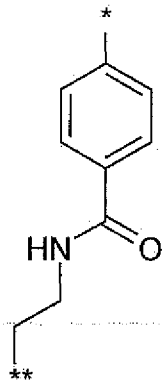 |
| 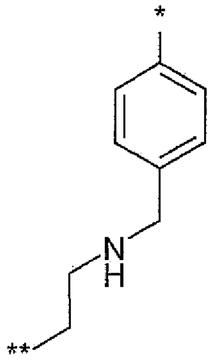 | $*-CH_2-[CH_2]_m-**$<br>m = 1-3 | 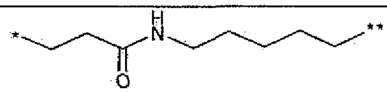 |

* representing the bond with the photoactive group X

** representing the bond with the ester group bonded to the polymer PS (PS-O-CO- group).

**20.** Process according to one of Claims 14 to 19, **characterized in that** the photoactive group X may be chosen from the following groups:

| | | | |
|---|---|---|---|
| $*-N_3$ | 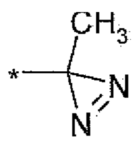 | 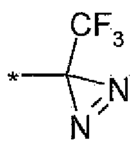 | 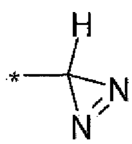 |

**21.** Process according to one of Claims 14 to 20, **characterized in that** the group X-L- is chosen from:

| | | |
|---|---|---|
| 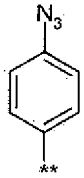 (i) | 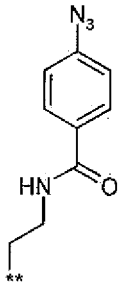 (ii) | 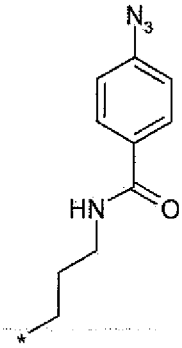 (iii) |
| $N_3$-$CH_2$-$CH_2$-** (iv) | 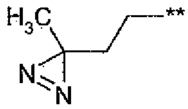 (v) | 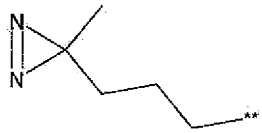 (vi) |
| 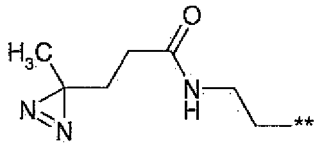 (vii) | 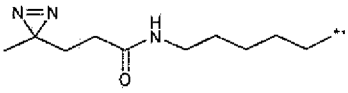 (viii) | 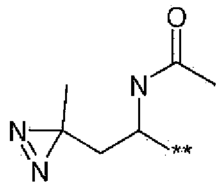 (ix) |
| 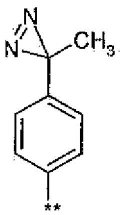 (x) | 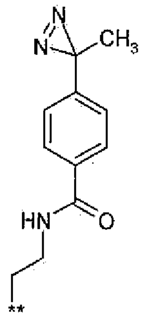 (xi) | 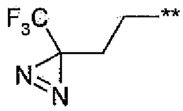 (xii) |
| 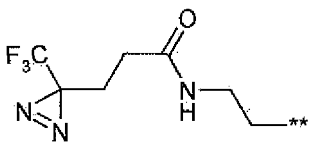 (xiii) | 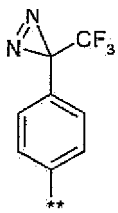 (xiv) | 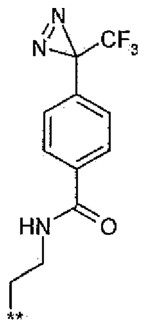 (xv) |
| ** representing the bond with the ester group bonded to the polymer PS (PS-O-CO- group) | | |

and preferably chosen from:

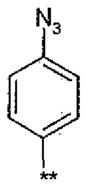

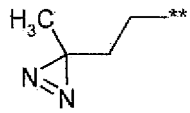

**22.** Process according to any one of Claims 14 to 21, **characterized in that** it has a weight-average molecular weight ranging from 5000 to 1 000 000 daltons, and preferably ranging from 10 000 to 500 000 daltons and preferentially ranging from 15 000 to 350 000 daltons.

**23.** Process according to any one of Claims 14 to 21, **characterized in that** the grafted polysaccharide (I) is present in a content ranging from 0.1% to 10% by weight, relative to the total weight of the composition, preferably ranging from 0.5% to 10% by weight, preferentially ranging from 1% to 8% by weight, and more preferentially ranging from 1% to 6% by weight.

**24.** Process according to any one of Claims 14 to 23, in which the step consisting in applying light radiation is performed after or at the same time as (simultaneously with) the step consisting in applying the cosmetic composition comprising the grafted polysaccharide (I).

**25.** Process according to any one of Claims 14 to 23, in which step (ii) consisting in applying light radiation is performed after step (i) consisting in applying the cosmetic composition comprising the grafted polysaccharide (I).

**26.** Process according to any one of Claims 14 to 25, **characterized in that** the light radiation is natural light or artificial light with a wavelength of between 360 and 600 nm.

**27.** Process according to one of Claims 14 to 25, in which the light radiation has a source chosen from arc lamps such as xenon lamps and mercury lamps; fluorescent lamps; incandescent lamps such as halogens; LEDs and lasers.

**28.** Process according to one of Claims 14 to 27, in which the exposure time to the light radiation is at least 5 seconds, and this exposure time may preferably range from 10 seconds to 15 minutes, especially between 15 seconds and 10 minutes and better still between 20 seconds and 5 minutes.

**29.** Process according to any one of Claims 14 to 28, **characterized in that** it is intended for attenuating wrinkles.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **AKIRA TAKAHASHI et al.** In situ cross-linkable hydrogel of hyaluronan produced via copper-free click chemistry. *Biomacromolecules,* 2013, vol. 14, 3581-3588
- **LAVILLE M et al.** Polysaccharide-covered nanoparticles with improved shell stability using click-chemistry stratégies. *Carbohydrate polymers,* 2013, vol. 93, 537-546